# EUROPÄISCHE PATENTSCHRIFT

(11) **EP 0 986 989 B1**
(45) Veröffentlichungstag und Bekanntmachung des Hinweises auf die Patenterteilung: **23.01.2002**
(21) Anmeldenummer: 98810930.2
(22) Anmeldetag: 17.09.1998
(51) Int. Cl.: A61B 17/32, F16C 1/02

(54) **Biegsame Welle aufweisendes chirurgisches Instrument**
Surgical instrument comprising flexible shaft
Instrument chirurgical comprenant un arbre flexible

(43) Veröffentlichungstag der Anmeldung: 22.03.2000
(73) Patentinhaber: Karl Storz GmbH & Co. KG, 78532 Tuttlingen (DE)
(72) Erfinder: Hiltebrandt, Siegfried, 75438 Knittlingen (DE)
(74) Vertreter: Lucht, Silvia, Dipl.-Phys.

(56) Entgegenhaltungen:
- EP-A- 0 445 918
- CH-A- 119 033
- DE-A- 4 002 449
- US-A- 4 646 738
- US-A- 5 488 761
- US-A- 5 512 007
- US-A- 5 669 926

## Beschreibung

Die Erfindung betrifft ein chirurgisches Instrument gemäss dem Oberbegriff des unabhängigen Patentanspruchs.

Instrumente der gattungsgemässen Art gibt es in unzähligen Ausführungsvarianten. Instrumente, wie sie auf dem Gebiet der Endoskopie und speziell auf dem Gebiet der Arthroskopie zum Einsatz kommen, weisen tpyischerweise ein Aussenrohr auf, welches in einem distalen Bereich, häufig im Bereich des distalen Endes des Aussenrohrs, eine Öffnung aufweist, in welcher Gewebe aufgenommen werden kann. In dem Aussenrohr ist ein Innenrohr angeordnet, welches in einem distalen Bereich, häufig am distalen Ende des Innenrohrs, mit einem Schneidwerkzeug versehen ist. Das Schneidwerkzeug kann direkt an dem Innenrohr angeformt sein, es kann aber auch ein separat herstellbares Teil sein, welches mit dem Innenrohr verbunden worden ist, z.B. durch Schweissen. Mit Hilfe des Schneidwerkzeugs kann am Ort des Einsatzes Gewebe abgetrennt werden, indem das Innenrohr relativ zum Aussenrohr rotiert. Dieses abgetragene Gewebe wird dann mit Hilfe von Unterdruck zusammen mit Spülflüssigkeit, die bei dieser Art der Operation normalerweise zum Einsatz kommt, durch das Innenrohr abgesaugt.

Mit den typischen (geradlinig verlaufenden) Instrumenten, die auf dem Gebiet der Endoskopie und insbesondere auf dem Gebiet der Arthroskopie zum Einsatz kommen, ist es jedoch für den behandelnden Arzt manchmal schwer oder sogar unmöglich, das Schneidwerkzeug an den gewünschten Ort zu bewegen (z.B. an die Unterseite der Patella, an die oberen und unteren Bereiche der Femurkondylen, oder an Bereiche der mondsichelförmig verlaufenden Menisci, insbesondere deren Vorder- und Hinterhörner), an welchem das Schneiden des Gewebes erfolgen soll. Unter "Gewebe" soll dabei jede Art von Gewebe verstanden werden, also Weichgewebe, Gewebe mittlerer Härte (wie z.B. Knorpelgewebe), aber auch sehr hartes Gewebe (wie z.B. Knochengewebe). Entsprechend sollen unter dem Begriff "Schneiden" alle in diesem Gebiet üblichen Arten von Abtragen verstanden werden, also insbesondere Schneiden, Fräsen, etc..

Um das Vordringen an solche nicht oder bestenfalls nur schwer zugänglichen Orte zu erleichtern, stehen auch von der typischen geradlinigen Form abweichende Instrumente zur Verfügung, z.B. solche, bei denen das Instrument im distalen Bereich abgewinkelt ist. Mit Hilfe derartiger Instrumente ist es einfacher, an Orte zu gelangen, die mit den herkömmlichen geradlinig ausgeführten Instrumenten nur schwer oder gar nicht zugänglich sind.

Es ist unmittelbar einleuchtend, dass bei einem solchen nicht geradlinig ausgeführten Instrument der starre proximale Teil des Innenrohrs die Antriebskraft bzw. das Antriebsmoment ebenfalls auf das im distalen Bereich, vorzugsweise am distalen Ende, vorgesehene Schneidwerkzeug übertragen muss, jedoch ist dabei der nicht-geradlinige Übergangsbereich zwischen dem proximalen Bereich des Innenrohrs und dem Schneidwerkzeug zu überwinden. Mit anderen Worten, das Innenrohr muss die Antriebskraft bzw. das Antriebsmoment über einen nicht-geradlinigen Übergangsbereich hinweg auf das Schneidwerkzeug übertragen.

Hierzu ist beispielsweise in der EP-A-0,445,918 ein Instrument beschrieben, welches zwischen dem starren proximalen Bereich und dem distalen Bereich einen flexiblen Übergangsbereich aufweist. Dieser flexible Übergangsbereich ist derart ausgeführt, dass dort mehrere diskrete Öffnungen vorgesehen sind derart, dass der Durchmesser des Innenrohres abwechselnd in der horizontalen bzw. vertikalen Richtung - jeweils senkrecht zur Längsachse des Innnerohrs - reduziert ist, sodass zwischen diesen Bereichen lediglich noch Stege vorgesehen sind, wobei benachbarte Stege jedoch miteinander verbunden sind.

Die miteinander verbundenen Stege gewährleisten die Flexibilität des Innenrohres im Übergangsbereich, andererseit gestatten sie eine Übertragung von Kräften oder Momenten auf das distal angeordnete Schneidwerkzeug. Allerdings sind die Kräfte oder Momente, die mit einem derartig ausgebildeten flexiblen Übergangsbereich übertragen werden können, einigermassen begrenzt. Die Begrenzung der Kräfte oder Momente, die noch auf das Schneidwerkzeug übertragen werden können, ist auch Sinn und Zweck dieser Massnahme, denn die Stege dienen auch als Sollbruchstellen. Überschreiten also die auf das Schneidwerkzeug zu übertragenden Kräfte bzw. Momente einen vorgegebenen Grenzwert, so brechen die Stege. Dadurch wird verhindert, dass bei sehr grossen aufzubringenden Kräften bzw. Momenten das Schneidwerkzeug zersplittern kann und möglicherweise einzelne Bruchstücke des Schneidwerkzeugs am Ort des Eingriffs ins Gewebe gelangen können.

Aus der US-A-5,669,926 ist ein chirurgisches Instrument zum Entfernen von Gewebe mit einem Außenrohr und einem Innenrohr bekannt. Das Innenrohr weist einen flexiblen Bereich auf, der die Form einer Schraubenfeder hat. Um die Öffnungen zwischen den einzelnen Windungen des flexiblen Bereichs des Innenrohrs zu verschließen, ist eine Umhüllung aus Plastik in Form eines Schrumpfschlauchs vorgesehen.

Aus der CH-A-119033 ist eine biegsame Welle in Form eines schraubenlinienförmig gewundenen Bandes bekannt. Die Bandränder weisen eine Verzahnung mit rechteckigem Verlauf auf, so daß die Berührungslinie der Ränder keine durchgehenden sonder eine gebrochene Schraubenlinie ist.

Wie bereits angesprochen, sind die zu übertragenden Kräfte bzw. Momente begrenzt. Gerade bei Wechselbelastungen, wie sie beim oszillierenden

Betrieb (ständiger Wechsel der Drehrichtung des Innenrohrs relativ zum Aussenrohr) solcher Instrumente auftreten, kann es aber sehr schnell zu Brüchen der Stege kommen. Ein oszillierender Betrieb ist andererseits als Option für den Betrieb eines solchen Instruments ausserordentlich nützlich, weil man gerade bei Gewebe mittlerer Härte und bei hartem Gewebe sehr oft nicht genau vorhersagen kann, welche Drehrichtung zum Abtragen von Gewebe geeigneter ist; zum Teil hängt dies auch von der geometrischen Gestalt des abzutragenden Gewebes ab und von welcher Richtung her man an das abzutragende Gewebe gelangt. Man kann in einigen solcher Fälle mit einem oszillierenden Betrieb des Instruments ein Abtragen von Gewebe erreichen, was mit einem Betrieb in nur einer einzigen Drehrichtung nicht so einfach oder gar nicht möglich wäre. Allerdings erfordert dies, dass die Wechselbelastungen von dem proximalen Bereich des Innenrohrs auf das Schneidwerkzeug übertragen werden können.

Es ist daher eine Aufgabe der Erfindung, ein Instrument vorzugschlagen, welches ein Innenrohr mit einem flexiblen Bereich aufweist, um auch für nicht-geradlinige Instrumente brauchbar zu sein, andererseits soll der flexible Bereich des Innenrohrs auch eine ausreichende Wechselfestigkeit aufweisen, um den Wechselbelastungen bei oszillierendem Betrieb standhalten zu können. Darüberhinaus soll das Innenrohr einfach in der Herstellung sein, insofern ist es auch eine Aufgabe der Erfindung, ein Rohr vorzugschlagen, welches einen flexiblen Bereich mit einer ausreichenden Wechselfestigkeit aufweist, um entsprechenden Wechselbelastungen standhalten zu können. Dabei soll das Innenrohr als solches natürlich gerade in dem flexiblen Bereich so dicht sein, dass abgesaugte Gewebeteile nicht aus dem Innenrohr austreten können.

Diese Aufgabe wird durch ein Instrument gelöst, welches die Merkmale des unabhängigen Patentanspruchs aufweist. Insbesondere handelt es sich dabei um ein chirurgisches Instrument zum Entfernen von Gewebe mit einem Aussenrohr, welches in einem distalen Bereich, vorzugsweise im Bereich des distalen Endes des Aussenrohrs, eine Öffnung zum Aufnehmen von Gewebe aufweist. Ferner umfasst das Instrument ein Innenrohr, welches innerhalb des Aussenrohrs angeordnet ist und einen starren proximalen Bereich aufweist, um Kräfte bzw. Momente, die auf diesen proximalen Bereich wirken, zu einem distalen Bereich des Innenrohrs zu übertragen, vorzugsweise zum distalen Ende des Innenrohrs. Ferner umfasst das Instrument ein Schneidwerkzeug, welches an dem distalen Bereich des Innenrohrs angeordnet ist, vorzugsweise am distalen Ende des Innenrohrs, um Gewebe schneiden zu können, welches im Bereich der Öffnung des distalen Bereichs des Aussenrohrs der Einwirkung des Schneidwerkzeugs ausgesetzt ist. Das Innenrohr weist zwischen seinem starren proximalen Bereich und dem Schneidwerkzeug einen flexiblen Bereich auf. In dem flexiblen Bereich weist das Innenrohr in seiner Wandung einen Schlitz auf, der sich in Längsrichtung des Innenrohrs betrachtet schraubenlinienförmig um die Längsachse des Innenrohrs herum windet und der entlang dieser Schraubenlinine betrachtet mäanderförmig verläuft. Durch diese Ausgestaltung kann die erforderliche Wechselfestigkeit, aber eben auch die notwendige Flexibilität, erreicht werden. Die Begriffe "Schneiden" und "Schneidwerkzeug" sollen - wie bereits früher erläutert - so verstanden werden, dass sie sämtliche in diesem Gebiet üblichen Arten von Abtragen von Gewebe erfassen, also insbesondere schneiden, fräsen, etc.. bzw. entsprechende Werkzeuge, also Schneidwerkzeuge, Fräswerkzeuge, etc..

Bei dem Instrument sind durch den mäanderförmig verlaufenden Schlitz abwechselnd Zähne und Einbuchtungen definiert, wobei in jeder Einbuchtung wiederum ein Zahn angeordnet ist und jeder Zahn in einer Einbuchtung angeordnet ist. Die Zähne und Einbuchtungen weisen eine Gestalt auf, die ein axiales Herausgleiten eines Zahnes aus einer Einbuchtung unmöglich macht. Dies ermöglicht, dass auch bei Instrumenten mit einem abgewinkelten distalen Bereich, bei denen der flexible Bereich zwangsläufig in axialer Richtung auseinandergezogen wird, die Zähne bei einer Drehbewegung des Innenrohrs stets in Eingriff mit der entprechenden Einbuchtung sind, sodass die Kräfte bzw. Momente sicher auf das Schneidwerkzeug übertragen werden.

Ein weitere Ausgestaltung zeichnet sich dadurch aus, dass die im Aussenrohr vorgesehene Öffnung zur Aufnahme des Gewebes im distalen Endbereich des Aussenrohrs angeordnet ist und das Schneidwerkzeug am distalen Ende des Innenrohrs angeordnet ist. Prinzipiell kann die Öffnung nämlich auch an einem anderen Ort als am distalen Ende des Aussenrohrs vorgesehen sein. Der Fall, bei welchem die Öffnung am distalen Ende des Aussenrohrs vorgesehen ist, ist jedoch der häufigste, denn man möchte ja das Instrument - wenn möglich - so wenig tief wie möglich in den Körper des Patienten bzw. in dessen Gelenk einbringen.

Die Breite des Schlitzes in der Wandung des Innenrohrs kann beispielsweise im Bereich von etwa 0.05 mm bis etwa 1 mm liegen, und die Wandstärke des Innenrohrs im Bereich von etwa 0.1 mm bis etwa 0.7 mm, insbesondere im Bereich von etwa 0.15 mm bis etwa 0.5 mm.

Die Steigung der Schraubenlinie, entlang welcher der in der Wandung des Innenrohrs vorgesehene Schlitz verläuft, kann beispielsweise im Bereich von etwa 0.5 mm/Windung bis etwa 4 mm/Windung liegen.

Das Schneidwerkzeug kann als separat herstellbares Element ausgebildet sein, welches mit dem distalen Ende des Innenrohrs verbunden ist. Dadurch ist es möglich, einerseits eine separate Herstellung von Schneidwerkzeug und Innenrohr zu ermöglichen, was den Herstellungsprozess erleichtert, andererseits können Schneidwerkzeug und Innenrohr zuverlässig miteinander verbunden werden, z.B. durch Schweissen, sodass die Übertragung der Kräfte bzw. Momente auf das Schneidwerkzeug gewährleistet ist. Alternativ kann das Schneidwerkzeug und das Innenrohr auch einstückig, also aus einem Stück, hergestellt sein.

Bei einem weiteren Ausführungsbeispiel kann das Aussenrohr im proximalen Bereich geradlinig verlaufen, während der distale Bereich des Aussenrohrs, in welchem das Schneidwerkzeug angeordnet ist, von dieser vom proximalen Bereich festgelegten geraden Linie abweichend ausgebildet ("abgewinkelt"). Das Innenrohr ist dann so ausgebildet, dass der flexible Bereich des Innenrohrs in einem sich zwischen dem proximalen Bereich und dem distalen Bereich des Aussenrohrs erstreckenden Übergangsbereich zu liegen kommt.

Dabei kann der distale Bereich des Aussenrohrs für sich alleine betrachtet ebenfalls geradlinig ausgebildet sein. Der flexible Bereich des Innenrohrs kommt in dem Übergangsbereich, in welchem der proximale Bereich und der distale Bereich ineinander einmünden, zu liegen.

Ein weitere unabhängiger Aspekt der Erfindung betrifft ein Rohr (z.B. das im Zusammenhang mit dem Instrument bereits erwähnte Innenrohr) mit einem starren proximalen Bereich und einem distal zu dem starren proximalen Bereich angeordneten flexiblen Bereich. Das Rohr weist in dem flexiblen Bereich in seiner Wandung einen Schlitz auf, der sich in Längsrichtung des Rohrs betrachtet schraubenlinienförmig um die Längsachse des Rohrs herum windet und der entlang dieser Schraubenlinine betrachtet mäanderförmig verläuft. Die Vorteile des Rohrs entsprechen den bereits anhand des Instruments gennanten Vorteilen (Wechselfestigkeit, Dichtigkeit, sichere Übertragung der erforderlichen Kräfte bzw. Momente).

Durch den mäanderförmig verlaufenden Schlitz sind abwechselnd Zähne und Einbuchtungen definiert, wobei in jeder Einbuchtung wiederum ein Zahn angeordnet ist und jeder Zahn in einer Einbuchtung angeordnet ist und die Zähne und Einbuchtungen eine Gestalt aufweisen, die ein axiales Herausgleiten eines Zahnes aus einer Einbuchtung unmöglich macht.

Die Breite des Schlitzes in der Wandung des Rohrs kann im Bereich von etwa 0.05 mm bis etwa 1 mm liegen, die Wandstärke des Rohrs im Bereich von etwa 0.1 mm bis etwa 0.7 mm, insbesondere im Bereich von etwa 0.15 mm bis etwa 0.5 mm.

Die Steigung der Schraubenlinie, entlang welcher der in der Wandung des Innenrohrs vorgesehene Schlitz verläuft, kann im Bereich von etwa 0.5 mm/Windung bis etwa 4.0 mm/Windung liegen.

Im folgenden wird die Erfindung anhand der Zeichnung näher erläutert. Es zeigen, teilweise in schematischer Darstellung und/oder im Schnitt:
- Fig. 1: ein Ausführungsbeispiel eines erfindungsgemässen chirurgischen Instruments in zusammengebautem Zustand,
- Fig. 2: den Ausschnitt II des flexiblen Bereichs des Innenrohrs aus Fig. 1,
- Fig. 3: den Ausschnitt III des flexiblen Bereichs aus Fig. 2,
- Fig. 4: einen Ausschnitt aus einem anderen Ausführungsbeispiel des flexiblen Bereichs
und
- Fig. 5: den Ausschnitt V aus dem flexiblen Bereich der Fig. 4.

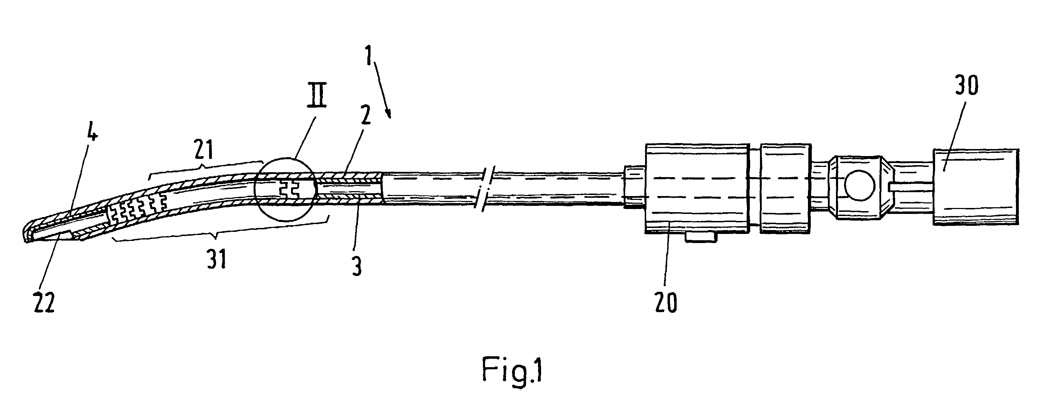

In Fig. 1 ist ein Ausführungsbeispiel eines erfindungsgemässen chirurgischen Instruments 1. Man erkennt, dass das Instrument 1 ein Aussenrohr 2 aufweist, in welchem ein Innenrohr 3 drehbar angeordnet ist. Das chirurgische Instrument 1 kann in einem Handstück (nicht dargestellt) aufgenommen werden, in welchem ein Drehantrieb, beispielsweise ein Elektromotor, vorgesehen sein kann zum rotatorischen Antreiben des Innenrohrs 3. Zur Ankopplung an den Drehantrieb ist das Innenrohr 3 an seinem proximalen Ende fest mit einem Kupplungsstück 30 verbunden, welches mit dem Drehantrieb in Eingriff gebracht werden kann. Das Aussenrohr 2 ist an seinem proximalen Ende fest mit einem Arretierungsstück 20 verbunden, welches von dem (nicht dargestellten) Handstück aufgenommen und dann in diesem arretiert wird. Das Kupplungsstück 30 und das mit diesem verbundene Innenrohr 3 sind relativ zu dem Arretierungsstück 20 und dem mit diesem verbundenen Aussenrohr 2 verdrehbar.

Im weiteren Verlauf vom proximalen Ende her beginnend ist das Innenrohr im proximalen Bereich starr ausgebildet bis hin zu einem flexiblen Bereich 31.
Dieser flexible Bereich 31 des Innenrohrs 3 erstreckt sich über einen gekrümmten Bereich 21 des Aussenrohrs 2 hinweg im wesentlichen bis hin zu einem Schneidwerkzeug 4, welches mit dem distalen Ende des Innenrohrs 3 verbunden ist. Das Schneidwerkzeug 4 kann als separat herstellbares Element ausgebildet sein, welches dann mittels einer geeigneten Verbindungstechnik, beispielsweise durch Schweissen, mit dem Innenrohr 3 verbunden wird. Alternativ kann das Schneidwerkzeug 4 und das Innenrohr 3 einstückig, also aus einem Stück, hergestellt sein. Unter "schneiden" bzw. "Schneidwerkzeug" sollen dabei - wie bereits früher mehrfach erläutert - alle in diesem Gebiet üblichen Arten von Abtragen verstanden werden, wie z.B. schneiden, fräsen, etc. bzw. die entsprechenden Werkzeuge, z.B. Schneidwerkzeuge, Fräswerkzeuge, etc.. Das Schneidwerkzeug 4 kann Gewebe abtragen, welches durch eine Öffnung 22 am distalen Ende des Aussenrohrs 2 aufgenommen wird. Das auf diese Weise abgetragene Gewebe kann dann durch den Innenraum des Innenrohrs 3 abgesaugt werden. Dabei ist sichergestellt, wie noch erläutert wird, dass abgetragenes und in das Innenrohr 3 eingesaugtes Gewebe nicht aus dem Innenraum des Innenrohrs 3 gelangen kann. Ferner ist durch die Art und Weise, wie der flexible Bereich 31 ausgebildet ist, sichergestellt, dass auch bei oszillierendem Betrieb die dabei auftretenden Kräfte und Momente sicher auf das Schneidwerkzeug 4 übertragen werden können, der flexible Bereich 31 weist also die dafür notwendige Wechselfestigkeit auf.

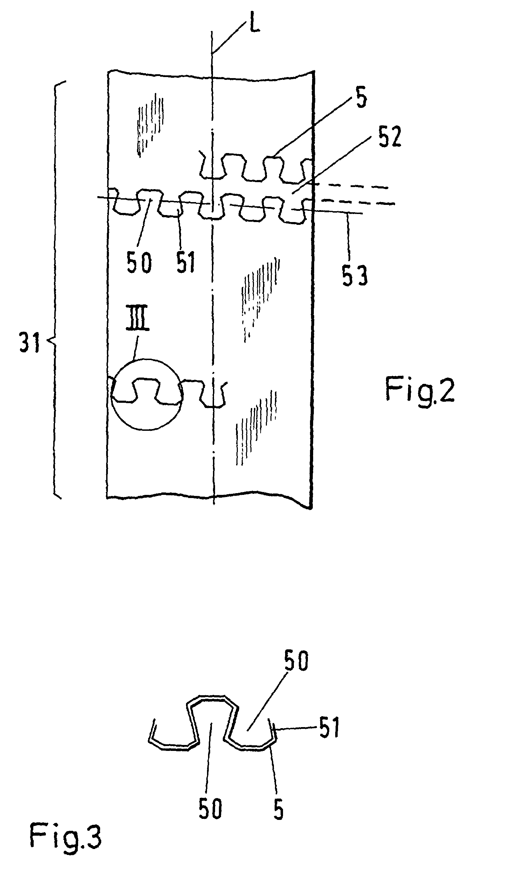

Fig. 2 zeigt den Ausschnitt II des flexiblen Bereichs 31 des Innenrohrs 3 in vergrösserter, abgewickelter Darstellung, Fig. 3 zeigt einen Ausschnitt aus Fig. 2 vergrössert. Man erkennt (Fig. 2), dass in der Wandung des Innenrohrs 3 ein Schlitz 5 vorgesehen ist, der sich schraubenlinienförmig um die Längsachse L des Innenrohrs 3 herum windet (der Schlitz ist nicht im gesamten Ausschnitt in Fig. 2 zeichnerisch dargestellt, sondern nur teilweise, er ist aber über den gesamten Bereich 31 vorhanden). Entlang der Schraubenlinie betrachtet verläuft dabei der Schlitz 5 mäanderförmig. Dabei sind durch den mäanderförmigen Verlauf des Schlitzes 5 abwechselnd Zähne 50 und Einbuchtungen 51 definiert. In jede durch den Schlitz 5 gebildete Einbuchtung 51 hinein ragt allerdings wieder ein Zahn 50 hinein. Betrachtet man zwei übereinander angeordnete Schlitze, so befindet sich zwischen den beiden Schlitzen ein Steg 52. Von diesem Steg stehen in axialer Richtung betrachtet die Zähne abwechselnd nach oben bzw. nach unten ab und ragen in entsprechende Einbuchtungen 51 hinein. Dabei weisen die einzelnen Zähne 50 und Einbuchtungen 51 eine Gestalt auf, die ein axiales Herausgleiten eines Zahnes 50 aus einer Einbuchtung 51 unmöglich macht.

Es ist nämlich klar, dass bei dem gekrümmten ("abgewinkelten") Instrument nach Fig. 1 der flexible Bereich 31 des Innerohrs 3 einer Beanspruchung auf Zug ausgesetzt ist. Der Schlitz 5 muss also so ausgebildet sein, dass die durch ihn gebildeten Zähne 50 und Einbuchtungen einerseits den gekrümmten Bereich 21 des Aussenrohrs 2 überwinden, andererseits eine Übertragung der erforderlichen Kräfte bzw. Momente auf das Schneidwerkzeug 4 ermöglichen, insbesondere also auch die erforderliche Wechselfestigkeit für oszillierenden Betrieb aufweisen.

Bei einem typischen Anwendungsbeispiel kann die Wandstärke der Wandung des Innenrohrs 3 im Bereich von etwa 0.1 mm bis etwa 0.7 mm liegen, insbesondere im Bereich von etwa 0.15 mm bis etwa 0.5 mm; die Breite des Schlitzes 5 kann im Bereich von etwa 0.05 mm bis etwa 1 mm liegen. Die Breite des Schlitzes kann dabei im Verlauf des Schlitzes durchaus variieren, sie muss also nicht in jedem Teilbereich des Schlitzes konstant sein. Die Schraubenlinie 53 des Schlitzes 5, entlang welcher der mäanderförmige Schlitz 5 verläuft, kann beispielsweise eine Steigung aufweisen, die im Bereich von etwa 0.5 mm/Windung bis etwa 4 mm/Windung liegt (ählich einer Gewindesteigung), insbesondere kann sie etwa 0.9 mm betragen. Der Steg 52 kann hier beispielsweise eine Breite von etwa 0.3 mm aufweisen. Das gesamte Innenrohr 3 kann einen Durchmesser von etwa 3 mm aufweisen. Selbstverständlich sind diese Werte nur als beispielhaft zu betrachten und können den jeweiligen Gegebenheiten bzw. Anforderungen angepasst werden.

Mit einem auf diese Weise ausgebildeten Innenrohr 3 können Gewebeteile, die mit Hilfe des Schneidwerkzeugs 4 abgetragen worden sind, zusammen mit einer Spülflüssigkeit durch den Innenraum des Innenrohrs 3 abgesaugt werden, ohne dass durch den Schlitz 5 hindurch Gewebeteile zwischen das Innenrohr 3 und das Aussenrohr 2 gelangen können, die ein Festfressen des Innenrohrs 3 im Aussenrohr 2 begünstigen könnten.

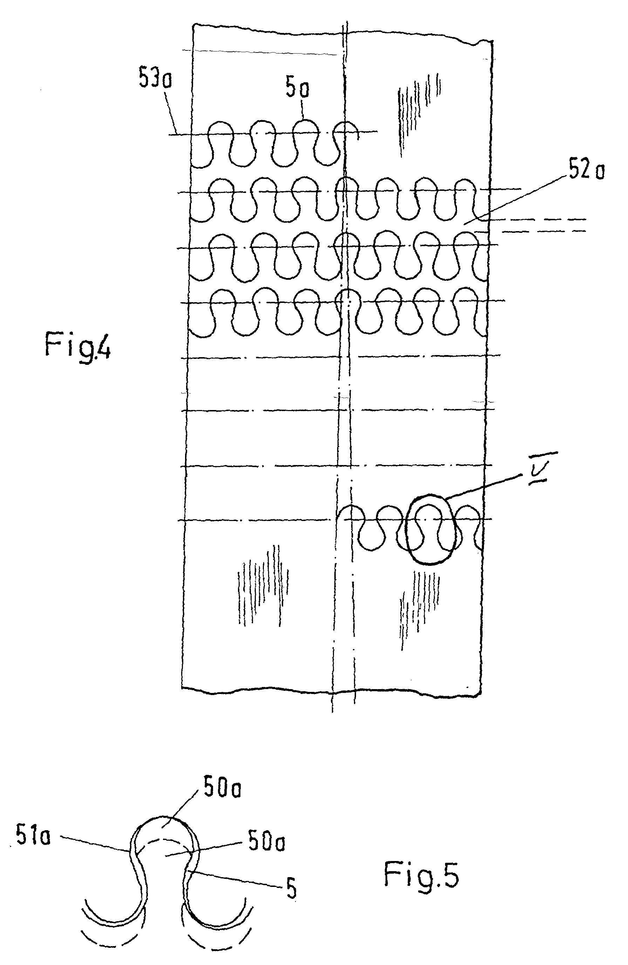

Ein anderes Ausführungsbeispiel des flexiblen Bereichs ist in Fig. 4 gezeigt, wieder in abgewickelter Darstellung, Fig. 5 zeigt vergrössert den Ausschnitt V aus Fig. 4. Die Bezugszeichen aus Fig. 3 und Fig. 4 sind beibehalten worden, es wurde ihnen lediglich der Buchstabe "a" hinzugefügt. Man erkennt demzufolge in Fig. 4 den Verlauf des Schlitzes 5a entlang der Schraubenlinie 53a, die hier allerdings eine andere Orientierung aufweist als in Fig. 3 (ähnlich einem Rechtsgewinde, während die Schraubenlinie 53 in Fig. 3 ähnlich einem Linksgewinde verläuft). Auch die Form der Zähne 50a und der Einbuchtungen 51 a hat sich von einer "eckigen" Ausgestaltung (Fig. 3 und Fig. 4) zu einer "abgerundeten" Ausgestaltung verändert. Gleich geblieben ist jedoch, dass ein Herausgleiten eines Zahnes 50a aus einer Einbuchtung 51 a nicht möglich ist. Dies ist besonders gut aus Fig. 5 ersichtlich, in welcher der Zahn 50a in der Einbuchtung 51a angeordnet ist, einmal ohne Zugbeanspruchung (durchgezogene Linie) und einmal mit Zugbeanspruchung (gestrichelte Linie). Im Falle der Zugbeanspruchung (gestrichelte Linie), dies ist bei Instrumenten mit einem zwischen dem proximalen und dem distalen Bereich liegenden gekrümmten Übergangsbereich des Aussenrohrs der Normalfall, liegt der Zahn 50a seitlich fest an der Einbuchtung 51a an, während er im Falle ohne Zugbeanspruchung (durchgezogene Linie) ein geringes Spiel (nämlich die Schlitzbreite an der jeweiligen Stelle) aufweist. Man erkennt in Fig. 5 auch gut, dass die Schlitzbreite entlang des Schlitzes 5a variieren kann. So ist der Schlitz im seitlichen Bereich deutlich breiter als am Kopfende.

Es ist klar, dass noch viele weitere Möglichkeiten des Verlaufs des mäanderförmigen Schlitzes möglich sind. Wesentlich ist, dass der jeweils dadurch gebildete Zahn nicht in axialer Richtung aus seiner zugehörigen Ausbuchtung herausgleiten kann. Die Gestalt der Zähne und der Einbuchtungen wie auch sonstige Parameter wie Steigung der Schraubenlinie, Stegbreite, Schlitzbreite und deren Variierung entlang des Schlitzes etc., können an die jeweiligen Anforderungen angepasst werden. Diese können sich insbesondere durch die Art des Gewebes ergeben, welches mit dem Schneidwerkzeug abgetragen werden soll (weiches Gewebe, gewebe mittlerer Härte, sehr hartes Gewebe). Es ist selbstverständlich, dass auch die Art des Abtragens (Schneiden, Fräsen, etc.) entsprechend dem jeweiligen Gewebe angepasst werden kann, indem ein Instrument mit einem entsprechenden Schneidwerkzeug gewählt wird.

## Patentansprüche

1. Chirurgisches Instrument (1) zum Entfernen von Gewebe
mit einem Außenrohr (2), welches in einem distalen Bereich, vorzugsweise im Bereich des distalen Endes des Außenrohrs (2), eine Öffnung (22) zum Aufnehmen von Gewebe aufweist,
und mit einem Innenrohr (3), welches innerhalb des Außenrohrs (2) angeordnet ist und einen starren proximalen Bereich aufweist, um Kräfte bzw. Momente, die auf diesen proximalen Bereich wirken, zu einem distalen Bereich des Innenrohrs (3) zu übertragen, vorzugsweise zum distalen Ende des Innenrohrs (3),
ferner mit einem Schneidwerkzeug (4), welches an dem distalen Bereich des Innenrohrs (3) angeordnet ist, vorzugsweise am distalen Ende des Innenrohrs (3), um Gewebe schneiden zu können, welches im Bereich der Öffnung (22) des distalen Bereichs des Außenrohrs (2) der Einwirkung des Schneidwerkzeugs (4) ausgesetzt ist,
wobei das Innenrohr (3) zwischen seinem starren proximalen Bereich und dem Schneidwerkzeug (4) einen flexiblen Bereich (31) aufweist,
wobei das Innenrohr (3) in dem flexiblen Bereich (31) in seiner Wandung einen Schlitz (5, 5a) aufweist, der sich in Längsrichtung des Innenrohrs (3) betrachtet schraubenlinienförmig um die Längsachse (L) des Innenrohrs (3) herumwindet, **dadurch gekennzeichnet, dass** der Schlitz (5,5a) entlang der Schraubenlinie (52, 52a) betrachtet meanderförmig verläuft, und
dass durch den mäanderförmig verlaufenden Schlitz (5, 5a) abwechselnd Zähne (50, 50a) und Einbuchtungen (51, 51a) definiert sind, wobei in jeder Einbuchtung (51, 51a) wiederum ein Zahn (50, 50a) angeordnet ist und jeder Zahn (50, 50a) in einer Einbuchtung (51, 51a) angeordnet ist und die Zähne und Einbuchtungen eine Gestalt aufweisen, die ein axiales Herausgleiten eines Zahnes (50, 50a) aus einer Einbuchtung (51, 51a) unmöglich macht.

2. Instrument nach Anspruch 1, **dadurch gekennzeichnet, dass** die im Aussenrohr (2) vorgesehene Öffnung (22) zur Aufnahme des Gewebes im distalen Endbereich des Aussenrohrs (2) angeordnet ist und das Schneidwerkzeug (4) am distalen Ende des Innenrohrs (3) angeordnet ist

3. Instrument nach einem der Ansprüche 1 bis 2, **dadurch gekennzeichnet, dass** die Breite des Schlitzes (5,5a) in der Wandung des Innenrohrs (3) im Bereich von etwa 0.05 mm bis etwa 1 mm liegt, und dass die Wandstärke des Innenrohrs (3) im Bereich von etwa 0.1 mm bis etwa 0.7 mm, insbesondere im Bereich von etwa 0.15 mm bis etwa 0.5 mm, liegt.

4. Instrument nach einem der vorangehenden Ansprüche, **dadurch gekennzeichnet, dass** die Steigung der Schraubenlinie (53,53a), entlang welcher der in der Wandung des Innenrohrs (3) vorgesehene Schlitz (5) verläuft, im Bereich von etwa 0.5 mm/Windung bis etwa 4 mm/Windung liegt.

5. Instrument nach einem der vorangehenden Ansprüche, **dadurch gekennzeichnet, dass** das Schneidwerkzeug (4) als separat herstellbares Element ausgebildet ist, welches mit dem distalen Ende des innenrohrs (3) verbunden ist.

6. Instrument nach einem der Ansprüche 1 bis 4, **dadurch gekennzeichnet, dass** das Schneidwerkzeug (4) und das Innenrohr (3) einstückig hergestellt sind.

7. Instrument nach einem der vorangehenden Ansprüche, **dadurch gekennzeichnet, dass** das Aussenrohr (2) im proximalen Bereich geradlinig verläuft, und **dass** der distale Bereich des Aussenrohrs (2), in welchem das Schneidwerkzeug (4) angeordnet ist, von dieser vom proximalen Bereich festgelegten geraden Linie abweichend ausgebildet ist, und dass das Innenrohr (3) so ausgebildet ist, dass der flexible Bereich (31) des Innenrohrs (3) in einem sich zwischen dem proximalen Bereich und dem distalen Bereich des Aussenrohrs (2) erstreckenden Übergangsbereich (21) zu liegen kommt.

8. Instrument nach Anspruch 7, **dadurch gekennzeichnet, dass** der distale Bereich des Aussenrohrs (2) für sich alleine betrachtet ebenfalls geradlinig ausgebildet ist und der flexible Bereich (31) des Innenrohrs (3) in dem Übergangsbereich (21), in welchem der proximale Bereich und der distale Bereich ineinander einmünden, zu liegen kommt.

## Claims

1. A surgical instrument (1) for removal of tissue
having an outer tube (2) that exhibits an opening (22) in a distal area, preferably in the area of the distal end of the outer tube (2) for accommodating tissue,
and having an inner tube (3) placed inside the outer tube (2) and that exhibits a rigid proximal area for the transfer of forces or moments acting on the said proximal area to a distal area of the inner tube (3), preferably to the distal end of the inner tube (3),
also equipped with a cutting tool (4) provided in the distal area of the inner tube (3), preferably in the distal end of the inner tube (3) in order to enable the cutting of tissue that is exposed to the action of the cutting tool (4) in the area of the opening (22) in the distal end of the outer tube (2),
in which the inner tube (3) exhibits a flexible area (31) between its rigid proximal area and the cutting tool (4),
in which the inner tube (3) exhibits a groove (5, 5a) in the wall of its flexible area (31) that, viewed in the longitudinal direction of the inner tube (3), winds in a spiral course around the longitudinal axis (L) of the inner tube (3),
**characterised in that** the groove (5, 5a) follows a meandering course when viewed along the spiral line (52, 52a), and
**in that** alternating teeth (50, 50a) and indentations (51, 51a) are defined by the meandering type groove (5, 5a), whereby one tooth (50, 50a) is situated in each indentation (51, 51a) in turn and every tooth (50, 50a) is situated in an indentation (51, 51a) and the shape of the teeth and indentations is such that it is impossible for a tooth (50, 50a) to slide axially out of an indentation (51, 51a).

2. An instrument in accordance with claim 1, **characterised in that** the opening (22) provided in the outer tube (2) for accommodating tissue is situated in the distal end area of the outer tube (2) and the cutting tool (4) is situated in the distal end of the inner tube (3).

3. An instrument in accordance with one of claims 1 to 2, **characterised in that** the width of the groove (5, 5a) in the wall of the inner tube (3) is within the range from approximately 0.05 mm to approximately 1 mm and that the thickness of the wall of the inner tube (3) is within the range from approximately 0.1 mm to approximately 0.7 mm, especially within the range from approximately 0.15 mm to approximately 0.5 mm.

4. An instrument in accordance with one of the preceding claims, **characterised in that** the gradient of the spiral line (53, 53a) along which the groove (5) provided in the wall of the inner tube (3) runs is within the range from approximately 0.5 mm/turn to approximately 4 mm/turn.

5. An instrument in accordance with one of the preceding claims, **characterised in that** the cutting tool (4) is developed as a separately producible component that is connected to the distal end of the inner tube (3).

6. An instrument in accordance with one of claims 1 to 4, **characterised in that** the cutting tool (4) and the inner tube (3) are produced as a single piece.

7. An instrument in accordance with one of the preceding claims, **characterised in that** the proximal area of the outer tube (2) is rectilinear and that the distal area of the outer tube (2) in which the cutting tool is situated (4) is developed in such a way that it deviates from this fixed straight line from the proximal area and that the inner tube (3) is developed in such a way that the flexible area (31) of the inner tube (3) is situated in a transitional area (21) extending between the proximal area and the distal area of the outer tube (2).

8. An instrument in accordance with claim 7, **characterised in that** the distal area of the outer tube (2) considered on its own is also rectilinear and the flexible area (31) of the inner tube (3) is situated in the transitional area (21) where the proximal area and the distal area join.

## Revendications

1. Instrument chirurgical (1) pour enlever du tissu,
doté d'un tube extérieur (2), qui présente dans une zone distale, de préférence dans la zone de l'extrémité distale du tube extérieur (2), une ouverture (22) pour le logement du tissu,
et d'un tube intérieur (3), qui est disposé à l'intérieur du tube extérieur (2) et présente une zone proximale rigide pour transmettre des forces et des couples, qui agissent sur cette zone proximale, à une zone distale du tube intérieur (3), de préférence à l'extrémité distale du tube intérieur (3),
doté également d'un outil de coupe (4) qui est disposé sur la zone distale du tube intérieur (3), de préférence sur l'extrémité distale du tube intérieur (3), pour pouvoir couper le tissu qui est exposé à l'effet de l'outil de coupe (4) dans la zone de l'ouverture (22) de la zone distale du tube extérieur (2), le tube intérieur (3) présentant une zone souple (31) entre sa zone proximale rigide et l'outil de coupe (4),
le tube intérieur (3) présentant dans la zone flexible (31) dans sa paroi une fente (5, 5a) qui s'entortille, vue dans le sens longitudinal du tube intérieur (3), autour de l'axe longitudinal (L) du tube intérieur (3) à la façon d'un hélicoïde, **caractérisé en ce que** la fente (5, 5a) s'étend à la façon d'un méandre vue le long de l'hélicoïde (52, 52a), **en ce qu'**alternativement des dents (50, 50a) et des creux (51, 51a) sont définis par la fente (5, 5a) disposée en forme de méandre, une dent (50, 50a) étant disposée à chaque fois dans chaque creux (51, 51a) et chaque dent (50, 50a) étant disposée dans un creux (51, 51a) et les dents et les creux présentant une forme qui rend impossible un glissement axial d'une dent (50, 50a) hors d'un creux (51, 51a).

2. Instrument selon la revendication 1, **caractérisé en ce que** l'ouverture (22) prévue dans le tube extérieur (2) pour le logement du tissu est disposée dans la zone d'extrémité distale du tube extérieur (2) et l'outil de coupe (4) sur l'extrémité distale du tube intérieur (3).

3. Instrument selon l'une quelconque des revendications 1 à 2, **caractérisé en ce que** la largeur de la fente (5, 5a) dans la paroi du tube intérieur (3) se situe dans la plage d'environ 0,05 mm à environ 1 mm, et **en ce que** l'épaisseur de paroi du tube intérieur (3) se situe dans la plage d'environ 0,1 mm à environ 0,7 mm, en particulier dans la plage d'environ 0,15 mm à environ 0,5 mm.

4. Instrument selon l'une quelconque des revendications précédentes, **caractérisé en ce que** la pente de l'hélicoïde (53, 53a), le long de laquelle s'étend la fente (5) prévue dans la paroi du tube intérieur (3), se situe dans la plage d'environ 0,5 mm / spire jusqu'environ 4 mm / spire.

5. Instrument selon l'une quelconque des revendications précédentes, **caractérisé en ce** l'outil de coupe (4) est conçu comme un élément qui peut être fabriqué séparément et est relié à l'extrémité distale du tube intérieur (3).

6. Instrument selon l'une quelconque des revendications 1 à 4, **caractérisé en ce que** l'outil de coupe (4) et le tube intérieur (3) sont fabriqués d'une seule pièce.

7. Instrument selon l'une quelconque des revendications précédentes, **caractérisé en ce** le tube extérieur (2) s'étend en ligne droite dans la zone proximale et en ce que la zone distale du tube extérieur (2), dans laquelle l'outil de coupe (4) est disposé, est conçue de façon différente de cette ligne droite définie par la zone proximale, et **en ce que** le tube intérieur (3) est conçu de façon que la zone flexible (31) du tube intérieur (3) vient s'appuyer dans une zone de transition (21) s'étendant entre la zone proximale et la zone distale du tube extérieur (2).

8. Instrument selon la revendication 7, **caractérisé en ce que** la zone distale du tube extérieur (2) est conçue de façon également rectiligne vue de façon individuelle et la zone flexible (31) du tube intérieur (3) vient s'appuyer dans la zone de transition (21), dans laquelle la zone proximale et la zone distale débouchent l'une dans l'autre.
